Europäisches Patentamt

European Patent Office

Office européen des brevets

(11) Veröffentlichungsnummer: **0 290 005**
**A2**

(12) **EUROPÄISCHE PATENTANMELDUNG**

(21) Anmeldenummer: **88107151.8**

(22) Anmeldetag: **04.05.88**

(51) Int. Cl.⁴: **C12N 15/00 , C12P 21/02 , C07K 3/12**

(30) Priorität: **06.05.87 DE 3715033**

(43) Veröffentlichungstag der Anmeldung:
**09.11.88 Patentblatt 88/45**

(84) Benannte Vertragsstaaten:
**AT BE CH DE ES FR GB GR IT LI LU NL SE**

(71) Anmelder: **Plückthun, Andreas, Dr.**
**Veit-Lung-Strasse 6**
**D-8033 Planegg(DE)**

Anmelder: **Glockshuber, Rudolf**
**Plassenburger Strasse 12**
**D-8000 München 90(DE)**

(72) Erfinder: **Plückthun, Andreas, Dr.**
**Veit-Lung-Strasse 6**
**D-8033 Planegg(DE)**
Erfinder: **Glockshuber, Rudolf**
**Plassenburger Strasse 12**
**D-8000 München 90(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) Verfahren zur Herstellung eines genetisch codierbaren Polypeptids.

(57) Unlösliche Fusionsproteine mit $\beta$-Galactosidase oder einem $\beta$-Galactosidase-Fragment von über 250 Aminosäuren lassen sich mit Harnstoff solubilisieren und können nach Dialyse unmittelbar der chemischen oder bevorzugt enzymatischen Spaltung zugeführt werden.

EP 0 290 005 A2

0 290 005

## Verfahren zur Herstellung eines genetisch codierbaren Polypeptids

Die Erfindung bezieht sich auf die gentechnische Herstellung von Polypeptiden über ein unlösliches Fusionsprotein mit einem Anteil der $\beta$-Galactosidase, wobei jedoch dieses Fusionsprotein nicht wie üblich in Suspension, sondern in Lösung der Spaltung zur Freisetzung des gewünschten Proteins zugeführt wird.

Verfahren zur Herstellung eines genetisch codierbaren Polypeptids durch Koppelung des Strukturgens für dieses Polypeptid im korrekten Leserahmen über ein Gen für $\beta$-Galactosidase an eine Regulationsregion, Einbringen dieser Genstruktur in ein Bakterium, Expression eines unlöslichen Fusionsproteins, Isolierung des Fusionsproteins nach einem Zellaufschluß und Gewinnung des gewünschten Polypeptids durch chemische oder enzymatische Spaltung sind in großer Zahl bekannt. Erfindungsgemäß wird nun das unlösliche Fusionsprotein unter besonderen Bedingungen wieder in Lösung gebracht, was nicht nur die Aufarbeitung erleichtert, sondern auch neue Möglichkeiten zur Aufarbeitung eröffnet.

K. Nagai et al., Proc. Natl. Acad. Sci. USA 82 (1985) 7252-7255; Nature 309 (1984) 810-812, haben bereits die Solubilisierung eines Fusionsproteins beschrieben. Allerdings handelt es sich hier um ein Fusionsprotein aus dem $\lambda$ cII-Protein und $\beta$-Globin, das relativ leicht löslich ist und nur durch eine hohe Salzkonzentration vorübergehend unlöslich gemacht wird.

Unerwarteterweise gelingt die Solubilisierung jedoch auch bei den extrem unlöslichen Fusionsproteinen, die erfindungsgemäß eingesetzt werden, wobei diese Proteine auch in Lösung bleiben. Man kann also einerseits die Vorteile nützen, die mit der hohen Stabilität der unlöslichen Fusionsproteine in der Wirtszelle verbunden sind, andererseits aber auch Aufarbeitungswege beschreiten, die ein lösliches Protein im Medium voraussetzen oder hierdurch sehr begünstigt werden.

Das erfindungsgemäße Verfahren ist dadurch gekennzeichnet, daß man das unlösliche Fusionsprotein, zweckmäßig bei einer Temperatur von 0°C bis Zimmertemperatur, vorzugsweise 4 bis 10°C, mit Harnstoff bis zur Solubilisierung versetzt, die Lösung vom Rückstand abtrennt, aus dieser Lösung den Harnstoff durch Dialyse entfernt und die so erhaltene Lösung des Fusionsproteins der Spaltung zuführt.

Im folgenden werden weitere vorteilhafte Ausgestaltungen der Erfindung näher erläutert.

Die untere Grenze der Harnstoffkonzentration ergibt sich durch die Solubilisierung des unlöslichen Fusionsproteins; sie liegt im allgemeinen bei etwa 6 M. Die obere Grenze ergibt sich aus der Löslichkeit des Harnstoffs im System; sie liegt bei etwa 8 M.

Die Menge des eingesetzten Fusionsproteins pro ml Harnstofflösung ist von den Löslichkeitseigenschaften des Fusionsproteins abhängig und kann bis 10 mg/ml betragen. In den meisten Fällen sind Fusionsprotein-Konzentrationen von etwa 1 bis etwa 2,5 mg/ml Harnstofflösung günstig.

Die Dialyse erfolgt bei Temperaturen von 0 bis 10°C, vorzugsweise 4°C, gegen eine übliche Pufferlösung. Der pH-Wert liegt im Bereich von 8 bis 9, vorzugsweise 8,3 bis 8,6. Auf einen Zusatz von Salzen wie Kochsalz wird zweckmäßig verzichtet.

Bei diesem Verfahren bleiben die Fusionsproteine in Lösung und werden bereits sehr stark angereichert, so daß sie im allgemeinen mit einer Reinheit über 75 % gewonnenen werden können.

Ein besonderer Vorteil des erfindungsgemäßen Verfahrens liegt darin, daß die so erhältlichen Proteinlösungen unmittelbar einer chemischen oder vorzugsweise enzymatischen Spaltung zugeführt werden können. Vorteilhaft ist beispielsweise die Spaltung mit Faktor Xa, die beispielsweise bei Nagai et al., a. a. O. oder in der europäischen Patentanmeldung mit der Veröffentlichungsnummer (im folgenden EP-A) 0 161 973 beschrieben ist. Dasselbe gilt für die Spaltung mit anderen Enzymen wie Trypsin oder IgA-Protease (aus Neisseria gonorrhoeae), da mit den erfindungsgemäß gewonnenen Proteinlösungen Bedingungen eingehalten werden können, bei denen keine Beeinträchtigung des gewünschten Proteins erfolgt.

Das erfindungsgemäße Verfahren bietet jedoch auch Vorteile für die chemische Spaltung von Fusionsproteinen, die in Lösung im allgemeinen schonender und schneller abläuft als in Suspension. So wird beispielsweise die Spaltung eines erfindungsgemäß eingesetzten Fusionsproteins der Prä-$\beta$-Lactamase mit Hydroxylamin sehr erleichtert.

Es zeigte sich überraschenderweise, daß das erfindungsgemäße Verfahren hinsichtlich der eingesetzten Fusionsproteine keinen Beschränkungen unterworfen ist. Offenbar spielt also der Charakter des gewünschten Proteins in dem eingesetzten Fusionsprotein keine entscheidende Rolle.

Das "Gen für $\beta$-Galactosidase" kann das unveränderte Wildtyp-Gen sein; im allgemeinen genügt aber ein verkürztes Gen, um ein hinreichend schwer lösliches Fusionsprotein zu erhalten. Solche verkürzten $\beta$-Galactosidase-Gene sind beispielsweise aus der EP-A 0 001 930 sowie aus Bröker, Gene Anal. Techn. 3 (1986) 53-57, bekannt. Besonders vorteilhaft sind Genkonstruktionen für ein $\beta$-Galactosidase-Fragment von über 250 Aminosäuren, jedoch signifikant weniger als die gesamte $\beta$-Galactosidase-Sequenz, vorteilhaft Genstrukturen, die für ein Fragment bis etwa 800, insbesondere bis etwa 650 Aminosäuren codieren.

2

Besonders günstig sind Genkonstruktionen, bei denen das β-Galactosidase-Fragment aus einer aminoterminalen und, oder einer carboxyterminalen Teilsequenz besteht.

Genkonstruktionen für eine verkürzte β-Galactosidase sind deshalb vorteilhaft, weil der "Ballast"-Anteil im zellfremden Protein geringer, die Ausbeute am gewünschten Protein also höher ist.

Die Figur 1 zeigt vorteilhafte verkürzte β-Galactosidase-Gene, wobei die mit A bezeichnete Reihe unmittelbar und die mit B bezeichnete Reihe über einen Linker an das Gen für das gewünschte Polypeptid gekoppelt werden.

Die Figur 2 zeigt die Konstruktion des Vektors pLZPWB (der die in Figur 1 unter A 2. aufgeführte Genkonstruktion enthält).

Die Figur 3 und ihre Fortsetzung, Figur 3 A, zeigen die Konstruktion der Vektoren pLZPWB/Xa/VL und pLZHP/Xa/VL A.

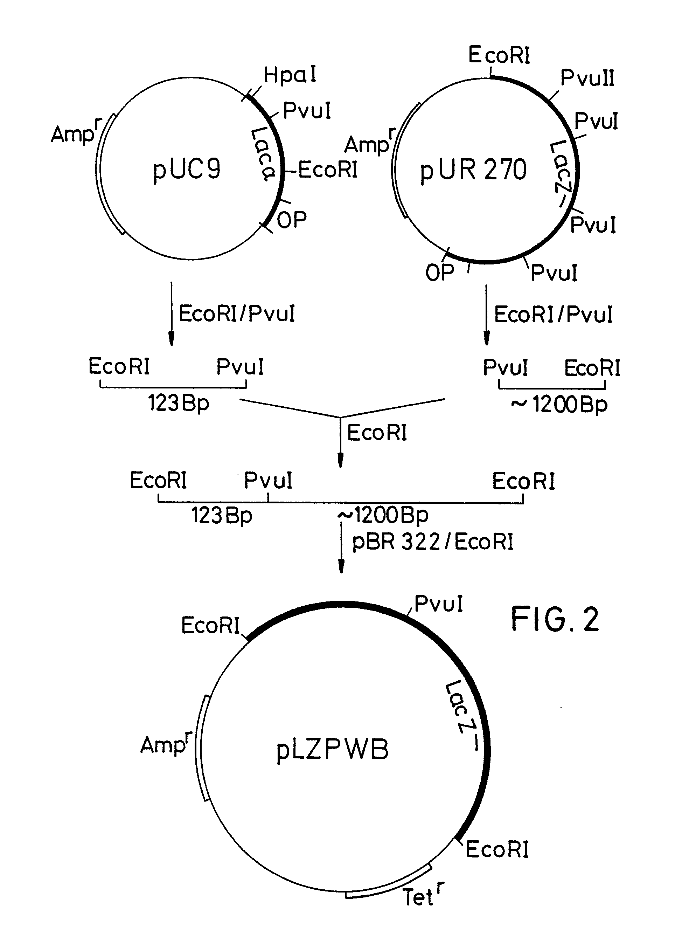

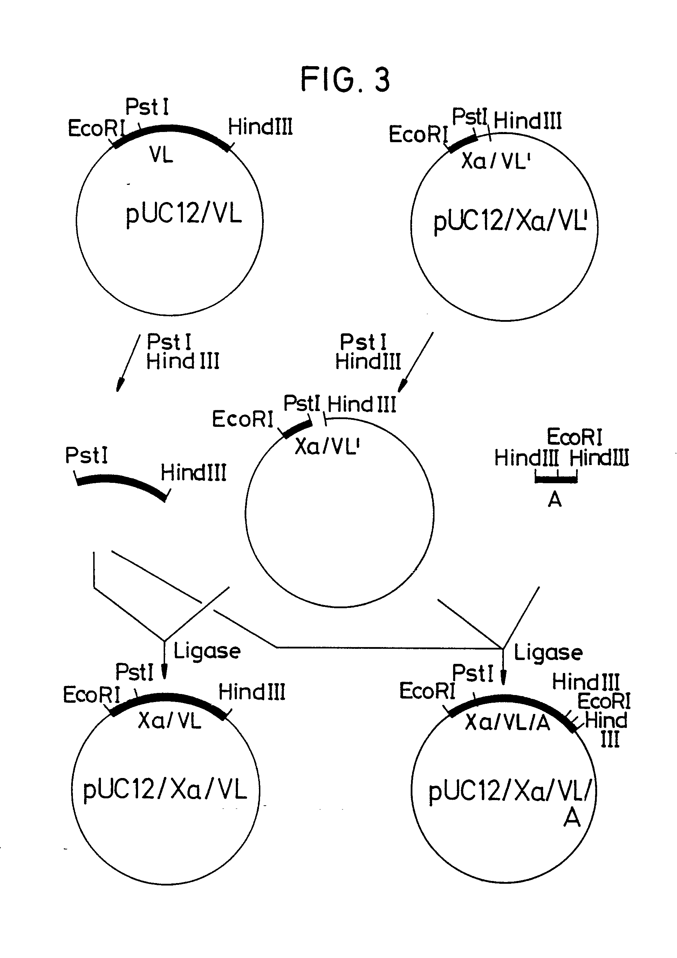

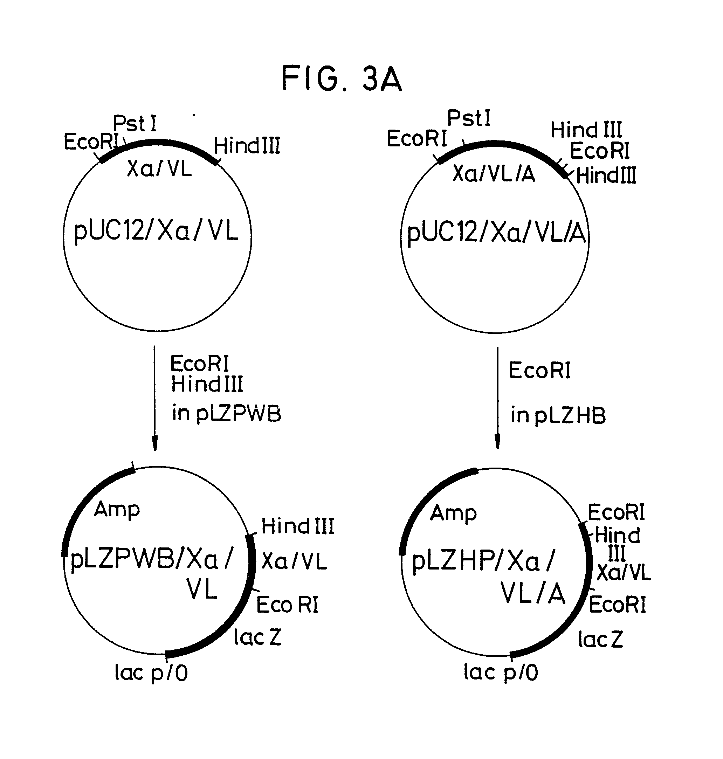

Die Figuren 2 und 3 sind zur Verdeutlichung nicht maßstäblich gezeichnet.

Eine vorteilhafte Ausgestaltung des erfindungsgemäßen Verfharens besteht darin, daß man zunächst das Gen für ein Fusionsprotein konstruiert, bei dem auf die DNA-Sequenz für das β-Galactosidase-Fragment von über 250 Aminosäuren ein DNA-Brückenglied folge, das für eine Aminosäure oder eine Gruppe von Aminosäuren codiert, die die chemische oder enzymatische Abspaltung des gewünschten Proteins ermöglicht, worauf das Strukturgen für das gewünschte Protein folgt. Dieses Gen für das Fusionsprotein wird in an sich bekannter Weise in einen E. coli-Expressionsvektor eingesetzt, in E. coli transformiert und dort das codierte Fusionsprotein zur Expression gebracht. Hierzu werden die Zellen nach Erreichen der gewünschten Konzentration, beispielsweise O.D.$_{550}$ = 1,0, mit Isopropyl-β-D-thiogalacto-pyranosid (IPTG) auf eine Endkonzentration von 1 mM für die erforderliche Zeit inkubiert und anschließend abzentrifugiert. Die Zellen werden dann in etwa 1/10 des ursprünglichen Volumens Natriumphosphatpuffer (50 mM, pH 7) resuspendiert, worauf die Zellsuspension in an sich bekannter Weise homogenisiert wird. Diese Homogenisierung kann beispielsweise durch eine zweimalige Passage durch eine French Press erfolgen. Geeignet ist auch eine Rührwerkskugelmühle, beispielsweise vom Typ [R]DYNO (Willi Bachofen, Basel).

Eine Lösung von 400 g Saccharose pro Liter des genannten Natriumphosphatpuffers vom vierfachen Volumen des Lysats wird dann mit dem Lysat überschichtet und zentrifugiert (SORVALL-Rotor GSA, 4000-13000 rpm, 4°C). Der Niederschlag von stark angereichertem Fusionsprotein wird in einem möglichst kleinen Volumen Tris-Puffer (10 mM, pH 8,3) aufgeschlämmt und ein relativ großes Volumen eines Tris-Puffers, vorteilhaft vom Volumen des ursprünglichen Lysats, der Harnstoff enthält (10 mM Tris-HCl, pH 8,3; 8 M Harnstoff), unter guter Durchmischung eingetragen, vorteilhaft unter Rühren eingetropft. Hierbei geht das Fusionsprotein in Lösung. Diese Harnstoff-Lösung wird erneut zentrifugiert (SORVALL-Zentrifuge, Rotor SS 34, 20000 rpm, 1 Stunde, 4°C). Der Überstand wird bei 4°C gegen Tris-Puffer (10 mM Tris-HCl, pH 8,3) dialysiert. Hierbei bleibt das Fusionsprotein in Lösung.

Das so angereicherte Fusionsprotein kann gewünschtenfalls in an sich bekannter Weise weiter gereinigt werden, was jedoch im allgemeinen nicht erforderlich ist. Es wird deshalb in den meisten Fällen die so erhaltene Proteinlösung unmittelbar der chemischen oder enzymatischen Spaltung zugeführt.

Die Erfindung wird durch die folgenden Beispiele näher erläutert. Prozentangaben beziehen sich hierbei auf das Gewicht, sofern keine anderen Angaben gemacht sind.


**Beispiel 1**


**DNA-Konstruktionen**


20 µg des handelsüblichen Plasmids pUC9 (vgl. Vieira et al., Gene 19 (1982) 259 - 268; The Molecular Biology Catalogue, Pharmacia P-L Biochemicals, 1984, Appendix, S. 40) werden einer Doppelverdauung mit den Restriktionsendonukleasen EcoRI und PvuI unterzogen und ein DNA-Fragment von 123 Basenpaaren (Bp) Länge gelelektrophoretisch abgetrennt. Dieses Fragment umfaßt einen Teil der aminoterminalen Codierungssequenz der β-Galactosidase.

Zur Isolierung des carboxyterminalen Anteils der β-Galactosidase bis zur natürlichen EcoRI-Schnittstelle werden 20 µg des Plasmids pUR270 (Rüther und Müller-Hill, EMBO J. 2 (1983) 1791-1794) zunächst mit EcoRI und anschließend mit dem Enzym PvuI verdaut. Durch Elektrophorese auf einem 5 %igen Polyacrylamidgel wird ein DNA-Fragment von ca. 1200 Bp abgetrennt und isoliert.

Die aminoterminalen und carboxyterminalen DNA-Fragmente der β-Galactosidase werden im Lauf von 6 Stunden bei 16°C zusammenligiert und das Ligierungsprodukt mit Ethanol gefällt. Die gefällte und resuspendierte DNA wird mit EcoRI geschnitten und noch einmal auf einem 5 %igen Polyacrylamidgel fraktioniert. Das DNA-Fragment der Länge ca. 1320 Bp wird durch Elektroelution aus dem Gel isoliert und in die Eco RI-Schnittstelle des Plasmids pBR322 ligiert. Das so erhaltene Hybridplasmid wird als pLZPWB bezeichnet.

Die vorstehend beschriebenen Reaktionsschritte sind in der Figur 2 dargestellt. Die Einzelmaßnahmen wurden in bekannter Weise (Maniatis et al., Molecular Cloning, Cold Spring Harbor 1982) durchgeführt.

Das Plasmid pLZPWB wird in E. coli transformiert, dort amplifiziert und reisoliert. Durch Verdauung mit EcoRI kann das amino-und carboxyterminal verkürzte $\beta$-Galactosidase-Genfragment herausgespalten und präparativ isoliert werden.

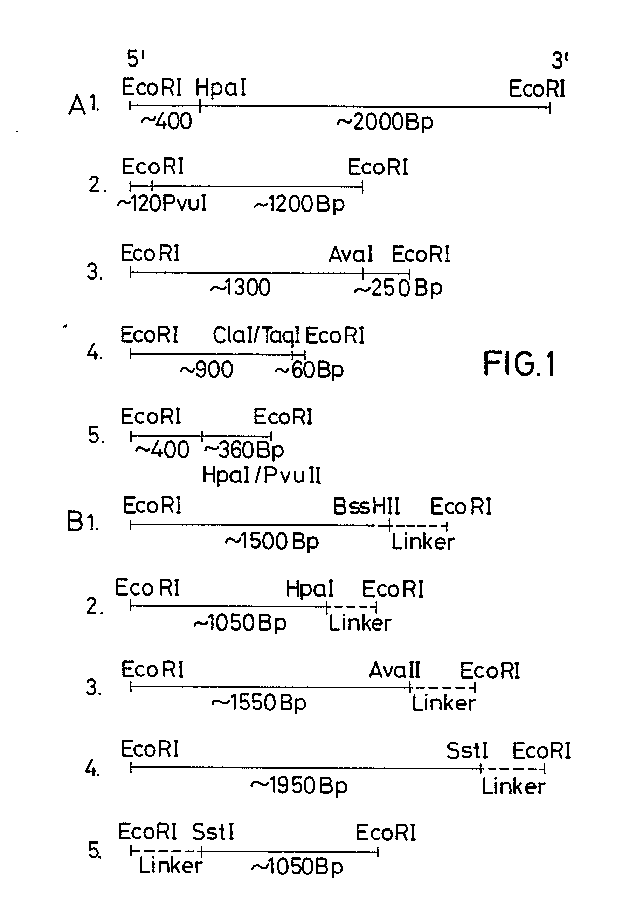

Über die bekannten Restriktionsenzym-Schnittstellen können weitere Verkürzungen konstruiert und in geeignete Expressionsplasmide eingefügt werden. Die Figur 1 A zeigt solche Verkürzungen. So wird der Vektor pLZHP erhalten, wenn analog zu dem beschriebenen Vorgehen einerseits pUC9 mit EcoRI und HpaI und andererseits pUR270 mit EcoRI und PvuII verdaut werden. Dieser Vektor enthält das Fragment A. 5 gemäß Figur 1.

Ähnliche Konstruktionen sind in analoger Weise möglich. Sowohl die Konstruktionen aus Figur 1, Teil A als auch die aus Figur 1 B sind so gewählt, daß der Leserahmen der verkürzten $\beta$-Galactosidase kontinuierlich übergeht in den Leserahmen des gewünschten carboxyterminalen Polypeptides. Die chemisch-synthetischen Linker der Figur 1, Teil B können beliebig gestaltet sein, müssen jedoch selbstverständlich den Stopcodon-freien Leserahmen gewährleisten und die gewünschten Restriktionsenzym-Schnittstellen aufweisen.

Das Gen für die variable Domäne der leichten Kette wurde ausgehend von der bekannten Proteinsequenz (Kabat, E.A. et. al., Sequences of Proteins of Immunological Interest, Nat. Inst. of Health (1983)) durch chemische Synthese entsprechend geplanter Oligonukleotide erhalten. Die vollständige DNA-Sequenz ist in Tabelle 1 wiedergegeben. In analoger Weise wurde die variable Domäne der schweren Kette synthetisiert (Tabelle 2).

Die Oligonukleotide wurden mit einem DNA-Synthesizer (Modell 380A von Applied Biosystems) mit der Phosphoramidit-Methode (Sinha, N.D. et. al., Nucleic Acid Res. 12 (1984) 4539) hergestellt. Die Oligonukleotide wurden über Polyacrylamid gereinigt und mit Polynukleotid-Kinase behandelt, hybridisiert und mit T4 Ligase ligiert (Dörper, T. und Winnacker, E.L., Nucleic Acid Res. 11 (1983) 2575 und Rommens, J. et al., Nucleic Acid Res. 11 (1983) 5921). Ein Fragment der erwarteten Größe wurde präparativ über Agarose-Gel-Elektrophorese gereinigt und in dem Vektor pUC12, der mit EcoRI und HindIII geschnitten war, ligiert. Die gesamte DNA-Sequenz wurde bestimmt und als korrekt verifiziert, sowohl für das Gen der leichten, als auch das der schweren Kette (Tabellen 1 bis 4).

Für die Konstruktion des Fusionsproteingens aus dem verkürzten $\beta$-Galactosidase-und dem $V_L$-Gen wurden zwei Oligonukleotide synthetisiert, die von der EcoRI-Stelle im lacZ-Gen des Vektors bis zur ersten singulären Schnittstelle im $V_L$-Gen, einer PstI-Stelle, reichen und dabei die Erkennungssequenz für die Protease Faktor Xa (Ile-Glu-Gly-Arg) einführen (Tabelle 3 und 4). Dabei wird das Start-Methionin, das im reifen Antikörper-Molekül nicht vorhanden ist, ebenfalls überflüssig. Die beiden genannten Oligonukleotide wurden zunächst im Vektor pUC12 zwischen der EcoRI-stelle und der PstI-Stelle des Polylinkers kloniert und durch DNA-Sequenzierung als fehlerfrei bestätigt (pUC12/Xa/$V_L'$; Fig. 3). Dieser Vektor wurde dann mit PstI und HindIII geschnitten und der restliche $V_L$-Anteil aus dem obengenannten pUC12-$V_L$-Plasmid als isoliertes PStI-HindIII-Fragment inseriert, um so das Plasmid pUC12/Xa/$V_L$ zu erhalten. Dieses Plasmid (pUC12/Xa/$V_L$) wurde mit EcoRI und HindIII geschnitten und das so erhaltene isolierte Fragment in den Vektor pLZPWB inseriert, der ebenfalls mit EcoRI und HindIII geschnitten wurde. Damit wurde das Ziel-Plasmid pLZPWB/Xa/$V_L$ erhalten. Die Korrektheit der Konstruktion wurde durch DNA-Sequenzierung belegt und die genaue Fusion ist in Tabelle 3 dargestellt.

In völlig analoger Weise wurde eine Fusion aus dem Vektor pLZHP und dem Gen der $V_L$-Region hergestellt. Dazu wurde das oben erwähnte PstI-HindIII-Fragment (das dem Hauptanteil des $V_L$-Gens entspricht) auch in Gegenwart eines HindIII-EcoRI-HindIII-Adapters A ligiert (Fig. 3 und deren Fortsetzung, Fig. 3A) und ergab des Vektor pUC12/Xa/$V_L$/A. Damit konnte das Gen mit der Faktor Xa-Erkennungssequenz auch mit EcoRI alleine herausgeschnitten werden und die EcoRI-Stelle des mit alkalischer Phosphatase behandelten Vektors pLZHP ligiert werden und ergab so das Plasmid pLZHP/Xa/$V_L$/A.

In völlig analoger Weise wurden auch Fusionen mit der synthetischen, variablen Domäne der schweren Kette desselben Antikörpers durchgeführt, sowohl im Vektor pLZPWB, als auch in pLZHP. Die Sequenz der Fusionsregion, beginnend mit der im lacZ-Gen befindlichen EcoRI-Stelle und der synthetischen Faktor Xa-Erkennungsstelle, ist in Tabelle 4 dargestellt.

**Beispiel 2**

**Herstellung, Isolierung und Spaltung der Fusionsproteine**

Jedes so erhaltene Plasmid wurde in den E. coli-Stamm W3110 transformiert. In einer typischen Präparation wurden die Zellen in 400 ml LB-Medium angezogen und bei einer $O.D._{550}$ von 1,0 mit IPTG (Endkonzentration 1 mM) induziert und über Nacht unter Schütteln inkubiert. Die Zellen wurden abzentrifugiert (SORVALL Rotor GSA, 4000 rpm, 15 min, 4°C) und in 40 ml Natriumphosphat-Puffer (50 mM, pH 7,0) resuspendiert. Die Zellen wurden durch zweimalige Passage durch eine French Press homogenisiert.

Eine Lösung (160 ml) von 400 g Saccharose pro Liter des genannten Natriumphosphat-Puffers wurden mit dem Lysat überschichtet und zentrifugiert (SORVALL Rotor GSA, 4000 rpm, 4°C, 30 min). Der Niederschlag wurde in 1 bis 2 ml Tris-Puffer (10 mM, pH 8,3) gründlich aufgeschlämmt und unter Rühren in 40 ml einer Harnstoff-Lösung eingetropft (10 mM Tris-HCl, pH 8,3, 8 M Harnstoff). Dabei ging das Fusionsprotein in Lösung. Diese Lösung wurde erneut zentrifugiert (SORVALL-Zentrifuge, Rotor SS34, 20000 rpm, 1 Stunde, 4°C). Der Überstand wurde bei 4°C gegen Tris-Puffer (10 mM Tris-HCl, pH 8,3) dialysiert. Die Lösung blieb klar; das Fusionsprotein blieb in Lösung.

Das so erhaltene Fusionsprotein wurde mit Faktor Xa aus Rinderblut gespalten. Der Faktor X wurde, wie von Fujikawa et al. (Fujikawa K., et al. Biochemistry 11 (1972) 4882) beschrieben, gereinigt und zu Faktor Xa aktiviert (Fujikawa K. et al., Biochemistry 11 (1972) 4982). Die Spaltung des Fusionsproteins mit Faktor Xa wurde bei 4°C in einem Tris-Puffer (50 mM Tris-HCl, pH 8,0, 100 mM NaCl, 1 mM $CaCl_2$) durchgeführt, in dem das Fusionsprotein auch löslich bleibt. Das Spaltprodukt wurde chromatographisch zur Homogenität gereinigt. Die korrekte Faktor Xa-Spaltung wurde durch Sequenzierung des N-Terminus des gereinigten Produktes bestätigt.

## Tabelle 1

Sequenz der variablen leichten Kette

```
        EcoRI                                              Pst I
        GAATTCCATGGATATCGTTATGACCCAGTCTCCGAGCTCTCTGTCTGTATCTGCAGGTGA
     1  ---------+---------+---------+---------+---------+---------+ 60
        CTTAAGGTACCTATAGCAATACTGGGTCAGAGGCTCGAGAGACAGACATAGACGTCCACT

                MetAspIleValMetThrGlnSerProSerSerLeuSerValSerAlaGlyGlu -

        ACGTGTTACCATGTCTTGCAAATCTTCTCAGTCTCTGCTGAACTCTGGTAACCAGAAAAA
    61  ---------+---------+---------+---------+---------+---------+ 120
        TGCACAATGGTACAGAACGTTTAGAAGAGTCAGAGACGACTTGAGACCATTGGTCTTTTT

                 ArgValThrMetSerCysLysSerSerGlnSerLeuLeuAsnSerGlyAsnGlnLysAsn -

        CTTCCTGGCGTGGTATCAGCAAAAGCCTGGCCAACCGCCGAAACTGCTGATCTACGGTGC
   121  ---------+---------+---------+---------+---------+---------+ 180
        GAAGGACCGCACCATAGTCGTTTTCGGACCGGTTGGCGGCTTTGACGACTAGATGCCACG

                 PheLeuAlaTrpTyrGlnGlnLysProGlyGlnProProLysLeuLeuIleTyrGlyAla -  .

        GTCGACCCGTGAATCTGGTGTTCCGGACCGTTTTACCGGTAGCGGTAGCGGTACCGACTT
   181  ---------+---------+---------+---------+---------+---------+ 240
        CAGCTGGGCACTTAGACCACAAGGCCTGGCAAAATGGCCATCGCCATCGCCATGGCTGAA

                 SerThrArgGluSerGlyValProAspArgPheThrGlySerGlySerGlyThrAspPhe -

        CACTCTGACCATCTCTTCTGTACAGGCTGAAGATCTGGCTGTTTACTACTGTCAAAACGA
   241  ---------+---------+---------+---------+---------+---------+ 300
        GTGAGACTGGTAGAGAAGACATGTCCGACTTCTAGACCGACAAATGATGACAGTTTTGCT

                 ThrLeuThrIleSerSerValGlnAlaGluAspLeuAlaValTyrTyrCysGlnAsnAsp -

        CCACTCTTACCCGCTGACCTTTGGCGCCGGCACCAAACTGGAACTGAAGCGCGCTTGATA
   301  ---------+---------+---------+---------+---------+---------+ 360
        GGTGAGAATGGGCGACTGGAAACCGCGGCCGTGGTTTGACCTTGACTTCGCGCGAACTAT

                HisSerTyrProLeuThrPheGlyAlaGlyThrLysLeuGluLeuLysArgAla****** -
        HindIII
        AGCTT
   361  ----- 365
        TCGAA
```

## Tabelle 2

Sequenz der variablen schweren Kette

```
      EcoRI
      GAATTCCATGGAAGTTAAACTGGTAGAGTCTGGTGGTGGTCTGGTACAGCCGGGTGGATC
    1 ---------+---------+---------+---------+---------+---------+ 60
      CTTAAGGTACCTTCAATTTGACCATCTCAGACCACCACCAGACCATGTCGGCCCACCTAG

            MetGluValLysLeuValGluSerGlyGlyGlyLeuValGlnProGlyGlySer -

      CCTGCGTCTGTCTTGCGCTACCTCAGGTTTCACCTTCTCTGACTTCTACATGGAGTGGGT
   61 ---------+---------+---------+---------+---------+---------+ 120
      GGACGCAGACAGAACGCGATGGAGTCCAAAGTGGAAGAGACTGAAGATGTACCTCACCCA

       LeuArgLeuSerCysAlaThrSerGlyPheThrPheSerAspPheTyrMetGluTrpVal -

      ACGTCAGCCCCCGGGTAAACGTCTCGAGTGGATCGCAGCTAGCCGTAACAAAGGTAACAA
  121 ---------+---------+---------+---------+---------+---------+ 180
      TGCAGTCGGGGGCCCATTTGCAGAGCTCACCTAGCGTCGATCGGCATTGTTTCCATTGTT

       ArgGlnProProGlyLysArgLeuGluTrpIleAlaAlaSerArgAsnLysGlyAsnLys -

      GTATACCACCGAATACAGCGCTTCTGTTAAAGGTCGTTTCATCGTTTCTCGTGACACTAG
  181 ---------+---------+---------+---------+---------+---------+ 240
      CATATGGTGGCTTATGTCGCGAAGACAATTTCCAGCAAAGTAGCAAAGAGCACTGTGATC

       TyrThrThrGluTyrSerAlaSerValLysGlyArgPheIleValSerArgAspThrSer -

      TCAATCGATCCTGTACCTGCAGATGAATGCATTGCGTGCTGAAGACACCGCTATCTACTA
  241 ---------+---------+---------+---------+---------+---------+ 300
      AGTTAGCTAGGACATGGACGTCTACTTACGTAACGCACGACTTCTGTGGCGATAGATGAT

       GlnSerIleLeuTyrLeuGlnMetAsnAlaLeuArgAlaGluAspThrAlaIleTyrTyr -

      CTGCGCGCGTAACTACTATGGCAGCACTTGGTACTTCGACGTTTGGGGTGCAGGTACCAC
  301 ---------+---------+---------+---------+---------+---------+ 360
      GACGCGCGCATTGATGATACCGTCGTGAACCATGAAGCTGCAAACCCCACGTCCATGGTG

       CysAlaArgAsnTyrTyrGlySerThrTrpTyrPheAspValTrpGlyAlaGlyThrThr -
                                                        HindIII
      CGTTACCGTTTCTTCTTGATAAGCTT
  361 ---------+---------+------ 386
      GCAATGGCAAAGAAGAACTATTCGAA

       ValThrValSerSer******
```

Tabelle 3

EcoRI

...GAATTCATCGAAGGTCGTGATATCGTTATG...
...CTTAAGTAGCTTCCAGCACTATAGCAATAC...

E  F  *I*  *E*  *G*  *R*  D  I  V  M

lac Z        Faktor Xa        V<sub>L</sub>

Tabelle 4

EcoRI

...GAATTCATCGAAGGTCGTGAAGGTAAACGT...
...CTTAAGTAGCTTCCAGCACTTCAATTTGAC...

E  F  *I*  *E*  *G*  *R*  E  V  K  L

lac Z        Faktor Xa        V<sub>H</sub>

Ansprüche

1. Verfahren zur Herstellung eines genetisch codierbaren Polypeptids durch Koppelung des Strukturgens für dieses Polypeptid im korrekten Leserahmen über ein Gen für β-Galactosidase an eine Regulationsregion, Einbringen dieser Genstruktur in ein Bakterium, Expression eines unlöslichen Fusionsproteins, Isolierung des Fusionsproteins nach einem Zellaufschluß und Gewinnung des gewünschten Polypeptids durch chemische oder enzymatische Spaltung, dadurch gekennzeichnet, daß man das unlösliche Fusionsprotein mit Harnstoff solubilisiert, aus dieser Lösung den Harnstoff durch Dialyse entfernt und die so erhaltene Lösung des Fusionsproteins der Spaltung zuführt.

2. Verfahren nach Anspruch 1, dadurch gekennzeichnet, daß die Solubilisierung bei einer Temperatur von 0°C bis Zimmertemperatur, vorzugsweise bei 4 bis 10°C erfolgt.

3. Verfahren nach Anspruch 1 oder 2, dadurch gekennzeichnet, daß die Harnstoffkonzentration bei etwa 6 bis etwa 8 M liegt.

4. Verfahren nach einem oder mehreren der vorhergehenden Ansprüche, dadurch gekennzeichnet, daß die Konzentration des Fusionsproteins in der Harnstofflösung bis 10 mg/ml beträgt.

5. Verfahren nach einem oder mehreren der vorhergehenden Ansprüche, dadurch gekennzeichnet, daß die Dialyse bei einer Temperatur von 0 bis 10°C, vorzugsweise bei etwa 4°C erfolgt.

6. Verfahren nach einem oder mehreren der vorhergehenden Ansprüche, dadurch gekennzeichnet, daß der pH-Wert der Dialyse-Lösung im Bereich von 8 bis 9, vorzugsweise 8,3 bis 8,6 liegt.

7. Verfahren nach einem oder mehreren der vorhergehenden Ansprüche, dadurch gekennzeichnet, daß die durch Dialyse angereicherte Lösung des Fusionsproteins ohne weitere Reinigung der chemischen oder vorzugsweise enzymatischen Spaltung zugeführt wird.

8. Verfahren nach einem oder mehreren der vorhergehenden Ansprüche, dadurch gekennzeichnet, daß das Gen für die β-Galactosidase für ein β-Galactosidase-Fragment von über 250 Aminosäuren, jedoch signifikant weniger als die gesamte β-Galactosidase-Sequenz, vorzugsweise bis etwa 800, insbesondere bis etwa 650 Aminosäuren, codiert.

9. Verfahren nach Anspruch 8, dadurch gekennzeichnet, daß das β-Galactosidase-Fragment aus einer aminoterminalen und/oder carboxyterminalen Teilsequenz besteht.

FIG.1

FIG. 2

# FIG. 3

# FIG. 3A

pUC12/Xa/VL

EcoRI  PstI  Xa/VL  HindIII

EcoRI
HindIII
in pLZPWB

pLZPWB/Xa/VL

Amp  HindIII  Xa/VL  EcoRI  lacZ  lac p/0

pUC12/Xa/VL/A

EcoRI  PstI  Xa/VL/A  HindIII  EcoRI  HindIII

EcoRI
in pLZHB

pLZHP/Xa/VL/A

Amp  EcoRI  Hind III  Xa/VL  EcoRI  lacZ  lac p/0